# EUROPÄISCHE PATENTSCHRIFT

(11) **EP 2 758 590 B1**
(45) Veröffentlichungstag und Bekanntmachung des Hinweises auf die Patenterteilung: **29.06.2016**
(21) Anmeldenummer: 12770418.7
(22) Anmeldetag: 18.09.2012
(51) Int. Cl.: D21C 1/04, D21C 3/04, D21C 5/00, D21C 7/14, C13K 1/02

(54) **VORRICHTUNG UND VERFAHREN ZUM HYDROTHERMALEN AUFSCHLUSS VON GETROCKNETEN, VERHOLZTEN LIGNOCELLULOSE-HALTIGEN BIOMASSEN**
DEVICE AND METHOD FOR HYDROTHERMALLY PULPING DRIED, LIGNIFIED LIGNOCELLULOSIC BIOMASS
DISPOSITIF ET PROCÉDÉ DE DÉCOMPOSITION HYDROTHERMIQUE DE BIOMASSES LIGNIFIÉES SÉCHÉES CONTENANT DE LA LIGNOCELLULOSE

(30) Priorität: 19.09.2011 DE 102011113646
(43) Veröffentlichungstag der Anmeldung: 30.07.2014
(73) Patentinhaber: Prüf- und Forschungsinstitut Pirmasens e.V., 66953 Pirmasens (DE)
(72) Erfinder: PACAN, Benjamin, 66957 Trulben (DE); DRÖGE, Stefan, 55232 Alzey (DE)
(74) Vertreter: Patentanwälte Dr. Keller, Schwertfeger
(86) Internationale Anmeldenummer: PCT/EP2012/003890
(87) Internationale Veröffentlichungsnummer: WO 2013/041207

(56) Entgegenhaltungen:
- WO-A1-2006/128304
- WO-A1-2010/080532
- WO-A1-2011/094859
- US-A- 6 090 595

## Beschreibung

### Technisches Gebiet:

Die vorliegende Erfindung betrifft eine Vorrichtung und Verfahren zum hydrothermalen Aufschluss von getrockneten, verholzten Lignocellulose-haltigen Biomassen, die vorzugsweise in Ballenform vorliegen.

Auf pflanzlichen Produkten basierende Biomassen stellen einen wichtigen Energie- und Rohstofflieferant zur Herstellung von Industrieprodukten oder Gewinnung von Biogas dar.

Die Ausweitung von Anbauflächen für Energiepflanzen, insbesondere für den Maisanbau, stößt jedoch vermehrt auf Akzeptanzprobleme, da mögliche negative Auswirkungen von Mais-Monokulturen und eine verstärkte Flächenkonkurrenz mit der Nahrungsmittelproduktion zunehmend die öffentliche Diskussion bestimmen. Zudem belasten die zuletzt deutlich gestiegenen Substratpreise den nachhaltig wirtschaftlichen Betrieb vieler laufender Biogasanlagen. Für die weitere Entwicklung der Biogaserzeugung wäre daher ein verstärkter Einsatz alternativer Rohstoffe dringend erforderlich.

### Stand der Technik:

Insbesondere Lignocellulose-haltige Biomassen, die allerdings in stark schwer abbaubarer, lignifizierter Form vorliegen, beispielsweise in Form von Stroh oder Heu, weisen ein hohes energetisches Potential auf. Solche Biomassen können als Substrat zur Nahrungsmittelproduktion oder Biogaserzeugung herangezogen werden, sofern sie verfahrenstechnisch aufbereitet werden. Im Stroh sind die Hemicellulose- und Cellulosefasern in eine äußerst stabile Ligninmatrix eingebettet, wodurch eine enzymatische Hydrolyse dieser Strukturpolymere und damit Weiterverarbeitung der Biomasse erheblich eingeschränkt ist. Deshalb sind Verfahren zur enzymatischen Hydrolyse solcher Biomassen, wie sie beispielsweise in der DE 2 643 800 C2 beschrieben sind, nur bedingt einsetzbar. Dabei ist die Nutzung solcher stark lignifizierten, verholzten Energiepflanzen für die Herstellung von Industrieprodukten (z.B. Ethanol oder Xylitol) oder zur Biogaserzeugung durchaus erstrebenswert.

Neben der starken Lignifizierung stehen darüber hinaus verfahrenstechnische Probleme einem Einsatz von unbehandeltem Stroh oder Heu entgegen. Dies betrifft insbesondere die Einbringtechnik sowie die ausgeprägte Schwimmdeckenbildung von strukturell intaktem Stroh. Eine wesentliche Voraussetzung für eine Nutzung von Stroh oder Heu für die Biogasproduktion ist daher die Entwicklung geeigneter Aufschlusstechnologien. Mechanische oder rein chemische Aufschlussverfahren erfordern einen relativ hohen Bedarf an elektrischer Energie oder verlangen einen deutlichen höheren und damit teuren Chemikalieneinsatz.

In der DE 10 2008 013 845 A1 wird ein Verfahren zur Herstellung von Bioethanol aus lignocellulosischer Biomasse beschrieben. Das Verfahren ist dazu vorgesehen, Kraftstoff-Ethanol aus einem Lignocellulose-Einsatzmaterial herzustellen. Dabei wird zunächst Cellulose und ggf. Hemicellulose aus der lignocellulosischen Biomasse gewonnen und anschließend zu Zuckern und im weiteren Prozessverlauf zu Bioethanol weiterverarbeitet. Das Verfahren ist dadurch gekennzeichnet, dass zerkleinerte lignocellulosische Biomasse mit einem Alkanolamin zur Extraktion des darin enthaltenen Lignins behandelt wird, die Lösung des Lignins abgetrennt und der anfallende Cellulose/Hemicellulose enthaltende Rückstand ohne Trocknen zu Zuckern umgesetzt und die Zucker schließlich zu Bioethanol fermentiert werden. Auch die EP 0 884 391 B1 beschreibt ein Vorbehandlungsverfahren für die Umwandlung von Cellulose in Kraftstoffethanol.

Grundsätzlich ist die Fermentation solcher Lignocellulose-haltiger Biomassen und insbesondere solcher in Ballenform nur eingeschränkt möglich, da die Vergärbarkeit des Substrates bei stark lignifizierten Biomassen sehr eingeschränkt ist. Die Biogaserträge der auf diese Weise fermentierten Lignocellulose-haltigen Biomassen sind daher vergleichsweise gering. In gepresster Form, beispielseise wenn das Ausgangssubstrat als Rest- oder Wickelballen vorliegt, ist die Fermentation besonders problematisch. Daher sind Fermentierungsverfahren, wie sie beispielsweise in der DE-OS 28 11 298 oder der DE-OS 37 17 725 beschrieben sind, nur bedingt für lignifizierte Biomassen einsetzbar und wirtschaftlich kaum zu betreiben. Die DE-OS 37 17 725 beschreibt zwar eine Vorrichtung zur Fermentierung von Strohballen, allerdings erfolgt die Fermentation unmittelbar in der darin beschriebenen Fermentationsvorrichtung. Ein vorheriger thermaler oder enzymatischer Aufschluss der Ballen ist nicht vorgesehen.

Die WO 2010/080532 A1 beschreibt ein Verfahren zur chemischen Vorbehandlung von cellulosehaltigen Materialien mit einer Säure. Die Säure wird mit dem Cellulosematerial in Verbindung gebracht und das Gesamtgemisch wird bei einer Temperatur im Bereich von vorzugsweise 160 bis 220° C erhitzt. Dabei muss die Biomasse zunächst mit der Säure vermengt und anschließend in einem Säure-/ Wassergemisch erhitzt werden, um eine Suspension zu bilden, die mit Dampf auf eine gewünschte Temperatur erhitzt und nach einer gewissen Verweilzeit an den atmosphärischen Druck angepasst wird.

Bei der WO 2011/094859 A1 ist ein Verfahren zur Verarbeitung von Lignocellulose-haltigem Material beschrieben, wobei das Material jedoch nicht in Ballenform vorliegt. Hierbei erfolgt eine Behandlung mit einer alkalischen Lösung bei einer Temperatur zwischen 140° C und ungefähr 250°C.

In der WO 2006/128304 A1 wird ein Verfahren zur Verarbeitung von Lignocellulose-haltigem Material beschrieben. Auch dies erfolgt unter Einsatz von Säuren in einem Reaktor bei einem pH zwischen ungefähr 0,4 und ungefähr 2,0. Anschließend erfolgt der Zusatz einer Base, um den pH schrittweise anzuheben.

Das US-Patent 6,090,595 A beschreibt ein ähnliches Verfahren zur Vorbehandlung von Lignocellulose-haltigem Material, welches zu Methanol umgewandelt werden soll. Auch hier erfolgt eine Hydrolyse der Cellulosen bei hohen Temperaturen.
Stark lignifizierte Restbiomassen wie Stroh fallen in Deutschland jedes Jahr in einem erheblichen Umfang an. Allein die Ernterückstände summieren sich, bezogen auf die Frischmasse, auf mehr als als 100 Mio to/a. Bezogen auf die Trockenmasse, die für eine stoffliche oder energetische Nutzung verfügbar ist, hat hierbei Getreidestroh das mit Abstand größte Potential. Stroh-liefernde Kulturarten wie Getreide, Mais und Ölsaaten werden in Deutschland auf schätzungsweise 8 Mio ha angebaut. Demnach ist Stroh, aber auch Heu, als Ernterückstand äußerst effizient nutzbar. Derzeit beschränkt sich die energetische Verwertung in erster Linie auf die Verbrennung von Stroh, die allerdings aufgrund der hohen Emission mit einigen Schwierigkeiten verbunden ist. Insofern stellt der Einsatz von Stroh als Substrat für Biogasanlagen eine interessante Alternative dar, da über den Gärrest der Lignin- und Mineralstoffanteil den Böden zurückgeführt werden kann.

Ein Verfahren, das geeignet ist, Lignocellulose-haltige Biomassen, die zu Ballenform gepresst sind, für die anschließende Weiterverarbeitung vorzubereiten, beispielsweise daraus gewonnene Hydrolysate einem Fermentationsprozess zuzuführen oder für die Biogaserzeugung einzusetzen, wäre wünschenswert.

### Darstellung der Erfindung:

Vor diesem Hintergrund ist es Aufgabe der vorliegenden Erfindung, eine Vorrichtung und ein verbessertes Verfahren zum Aufschluss von getrockneten, verholzten Lignocellulose-haltigen Biomassen, insbesondere solchen, die in Ballenform vorliegen, bereitzustellen, mit der/dem ein hoher Aufschlussgrad bei gleichzeitig geringem Chemikalieneinsatz möglich ist.

Diese Aufgabe wird gelöst durch eine Vorrichtung mit den Merkmalen des Anspruchs 1 und einem Verfahren mit den Merkmalen des Anspruchs 5. Bevorzugte Ausführungsformen finden sich in den Unteransprüchen wieder.

Die vorliegende Erfindung betrifft sowohl eine Vorrichtung zum hydrothermalen Aufschluss von getrockneten, verholzten Lignocellulose-haltigen Biomassen, die in Ballenform vorliegen, als auch ein Verfahren zum hydrothermalen Aufschluss solcher Biomassen.

Die erfindungsgemäße Vorrichtung zum hydrothermalen Aufschluss von getrockneten, verholzten Lignocellulose-haltigen Biomassen, die in Ballenform vorliegen, besteht aus einem Aufschlussbehälter zur Aufnahme eines oder mehrerer Biomasseballen mit einer, vorzugsweise an der Stirnseite des Behälters angeordneten, Beschickungsöffnung für die Biomasseballen sowie einer, vorzugsweise auf der gegenüberliegenden Stirnseite angeordneten, Entleerungsöffnung für die Entfernung der säurebehandelten Biomasse aus dem Aufschlussbehälter. Über eine Zuführleitung wird ein vorgeheiztes Säure/Wassergemisch über Füllstutzen am Behälter in das Innere des Aufschlussbehälters geleitet. Das beim hydrolytischen Aufschluss entstehende Hydrolysat ist über einen Hydrolysatablauf am Behälterboden aus dem Aufschlussbehälter abführbar und für die jeweiligen Anwendungen weiterverarbeitbar.

In dem Aufschlussbehälter befindet sich wenigstens eine stationäre oder bewegliche Injektionseinrichtung zur Einleitung des vorgeheizten Säure/Wassergemisches. Bei der stationären Ausführungsform wird der Behälter entweder mit dem Säure/Wassergemisch gefüllt oder die Biomasseballen von der Behälterdecke aus berieselt. In der beweglichen Ausführungsform ist die Injektionseinrichtung vorzugsweise vertikal verstellbar, sodass diese das Säure/Wassergemisch direkt in den Biomasseballen injizieren kann. Vorzugsweise ist die Injektionseinrichtung als Einstechlanze ausgebildet und wird über einen Führungszylinder von der Oberseite des Aufschlussbehälters in den Biomasseballen zur Injektion des Säure/Wassergemisches in den Ballen eingeschoben. Das Säure/Wassergemisch wird somit direkt in den Ballen injiziert, was den hydrothermalen Aufschluss erheblich begünstigt. Vorzugsweise befinden sich mehrere Injektionseinrichtungen an der Oberseite des Aufschlussbehälters, wobei diese vertikal verschiebbar sind. Haben die Ballen ihre Position in dem Aufschlussbehälter erreicht, so werden die Injektionseinrichtungen von oben nach unten gefahren, sodass sich die Spitze der Injektionseinrichtung in den Ballen bohrt. Über Zuführleitungen wird das vorgewärmte Säure/Wassergemisch dann in den Ballen geleitet.

Im Bodenbereich des Aufschlussbehälters ist ferner ein Sieb- und Führungselement zur Aufnahme der Biomasseballen in das Behälterinnere und zur Abtrennung des flüssigen Hydrolysats von der festen Biomasse angeordnet. Vorzugsweise handelt es sich hierbei um ein Lochblech.

Die erfindungsgemäße Vorrichtung hat den Vorteil, dass die Biomasse in gepresster Form, d.h. in Ballenform (z.B. gepresst als Rest- oder Wickelballen) mit einem hohen Dichtegrad direkt in den Aufschlussbehälter einführbar ist. Durch die Ballenform und der damit verbundenen hohen Dichte der darin enthaltenen Biomasse kann, bezogen auf den für einen Ballen benötigten Platzbedarf, ein großes Volumen an Gesamtbiomasse im Reaktionsbehälter behandelt werden. Nach dem Pressen und Transport der Ballen kann zudem eine zügige Weiterverarbeitung erfolgen.

Im Anschluss an die Beschickung des Aufschlussbehälters mit den Biomasseballen erfolgt der hydrothermale Aufschluss durch Behandlung der Biomasse mit einem Säure/Wassergemisch mineralischer und/oder organischer Säure(n). Der hydrothermale Aufschluss erfolgt vorzugsweise bei Temperaturen zwischen 120° und 220° C, wobei sich Temperaturen zwischen 140° und 160° C als besonders vorteilhaft erwiesen haben. Durch den Einsatz von Säuren bzw. Säurekatalysatoren lässt sich die Aufschlusstemperatur sehr niedrig halten, was eine deutliche Energieersparnis mit sich bringt.

Die Vorwärmung des Säure/Wassergemisches erfolgt vorzugsweise über eine Vorheizeinrichtung. Hierbei handelt es sich vorzugsweise um einen Wärmetauscher, dessen Energie beispielsweise von einem Blockheizkraftwerk stammen kann. Die Vorwärmung des Säure/Wassergemisches hat sich als besonders vorteilhaft erwiesen. Denkbar ist auch eine Erwärmung/Aufheizung des Aufschlussbehälters oder der Reaktionskammer; hierbei ist jedoch der Energiebedarf deutlich höher. Das Säure/Wassergemisch wird vorzugsweise auf eine Temperatur von 180° C erhitzt und dem Aufschlussbehälter über entsprechende Füllstutzen zugeführt. Im Aufschlussbehälter selbst beträgt die Temperatur der injizierten Flüssigkeit idealerweise dann etwa 140° C bis 160° C.

Damit der hydrothermale Aufschluss möglichst effizient abläuft und kontrolliert werden kann, umfasst die erfindungsgemäße Vorrichtung ferner Mittel zur Temperaturüberwachung und/oder Drucküberwachung und/oder pH-Überwachung und/oder ein Sicherheitsventil und/oder einen Dampfauslass. Diese Mittel und Einrichtungen ermöglichen einen effizienten und kontrollierten Verfahrensablauf, um einen möglichst hohen Aufschlussgrad der Biomasse und eine hohe Zuckerausbeute im Hydrolysat aus der bestückten Biomasse zu erzielen.

Das erfindungsgemäße Verfahren zum hydrothermalen Aufschluss von getrockneten, verholzten Lignocellulose-haltigen Biomassen, die vorzugsweise in Ballenform vorliegen, umfasst in einem ersten Schritt a. die Inkubation der Lignocellulose-haltigen verholzten Biomasse bei einer Temperatur zwischen 120° und 220° C mit einem vorgeheizten Säure/Wassergemisch, bestehend aus einer mineralischen und/oder organischen Säure. In einem sich anschließenden optionalen zweiten Schritt erfolgt, je nach Weiterverarbeitung, ein enzymatischer Aufschluss der in Schritt a. säurebehandelten Biomasse mit einem Gemisch aus cellulolytischen Enzymen bei einer Temperatur zwischen 35° und 65° C und einem Säuregrad (pH) im Bereich von 3 bis 6.

Für die Gewinnung von Xylose aus Lignocellulose-haltiger Biomasse zur fermentativen Gewinnung von Xylitol und die anaerobe Biogasgewinnung ist der zuvor beschriebene hydrothermale Aufschluss im Schritt a. ausreichend. Zur Xylitolfermentation ist lediglich eine geringe Glukosekonzentrationen für den Aufbau der Zellbiomasse erforderlich, weshalb das hydrothermal gewonnene Hydrolysat vor der Fermentation vom Hydrolyserest abgetrennt wird. Bei der anschließenden Verwertung des Hydrolyserestes zur Biogasgewinnung führt die weitere enzymatische Behandlung der Biomasse im Schritt b. zur Beschleunigung des Biogasbildungsprozesses. Für den Einsatz in konventionellen Biogasanlagen mit einer hydraulischen Verweilzeit von 60 bis 100 Tagen ist deshalb eine enzymatische Behandlung des Hydrolyserestes nicht unbedingt erforderlich.

Vorzugsweise findet die Inkubation der Lignocellulose-haltigen verholzten Biomasse bei einer Temperatur zwischen 140° und 160° C statt. Die Säurekonzentrationen in dem Säure/Wassergemisch können sehr gering gehalten werden. Bei der hydrothermalen Hydrolyse fungiert Wasser unter subkritischen Bedingungen (Temperatur zwischen 120° und 220° C und Druck zwischen 2 und 20 bar) als Lösungsmittel, wobei die natürlichen Inhaltsstoffe der verwendeten Biomasse unterstützend wirken. Die mit dem Temperaturanstieg sinkende Oberflächenspannung, eng gekoppelt an die Benetzungseigenschaften und die stark verringerte Dielektrizitätskonstante ermöglichen, dass subkritisches Wasser als Lösungsmittel für organische Stoffe einsetzbar ist. Bei den auftretenden hohen Temperaturen und Drücken wird die Hydrolyse von Lipiden, Proteinen und Kohlenhydratpolymeren, insbesondere von Hemicellulosen, beschleunigt. Daneben kann bei Temperaturen von 180° bis 200° C Lignin aus dem pflanzlichen Gewebe herausgelöst werden.

Die hydrothermale Behandlung von Stroh mit einem Säure/Wassergemisch mineralischer und/oder organischer Säuren bewirkt, gezeigt anhand des Aufschlusses von Stroh, eine hydrolytische Spaltung von Xylanen und anderen Hemicellulosen:

R1-O-R2 + H20 → R1OH + R2OH.

Ferner werden 50 % des in der Cellulose inkrustierten Lignins gelöst.

Für eine Abspaltung von Cellulose in monomere Zucker sind normalerweise Temperaturen von mehr als 200° C notwendig. Solch hohe Temperaturen erfordern einen hohen Energieeinsatz, weshalb der Celluloseabbau erst in einer weiteren Verfahrensstufe enzymatisch nach der hydrothermalen Verzuckerung der Hemicellulose und der teilweisen Herauslösung des Lignins erfolgen soll.

Erfindungsgemäß wird zur Absenkung der Aufschlusstemperatur und Erhöhung des Aufschlussgrades wenigstens ein mineralischer oder organischer Säurenkatalysator zugesetzt. Zur Absenkung des Temperaturniveaus und zur Erhöhung der Aufschlusseffizienz ist der Einsatz mineralischer Säuren besonders bevorzugt. Durch den Einsatz von Säuren lassen sich bei Temperaturen zwischen 140° und 150° C mehr als 75 % (bei optimalen Temperaturbedingungen mehr als 90 %) der Hemicellulosefraktion in Monosaccharide überführen. Somit ist neben der thermischen Behandlung auch der Einsatz mineralischer und/oder organischer Säuren unbedingt erforderlich.

Als Säure/Wassergemisch wird vorzugsweise eine mineralische Säure eingesetzt, ausgewählt aus der Gruppe bestehend aus Salpetersäure, Schwefelsäure, Salzsäure, Phosphorsäure, Milchsäure. Salpetersäure ist aufgrund der Wirtschaftlichkeit der Anwendung (geringe Edelstahlkorrosion, unproblematisch für Biogasprozess) besonders bevorzugt.

Die im erfindungsgemäßen Verfahren eingesetzten mineralischen Säuren werden vorzugsweise mit folgenden Konzentrationen eingesetzt:

**Tabelle 1: Einsatzbereiche mineralischer Säuren zum hydrothermalen Aufschluss lignifizierter Biomassen**

| **Säure** | **Konzentration [%]^{a)}** | **Menge je kg TM [ml/kg]** |
|---|---|---|
| Salpetersäure [HNO₃] | 0,1 - 0,7 | 13 - 90 ^{b)} |
| Schwefelsäure [H₂SO₄] | 0,1 - 0,5 | 9 - 60 ^{c)} |
| Salzsäure [HCl] | 0,1 - 0,5 | 22 - 160 ^{d)} |
| ortho-Phosphorsäure [H₃PO₄] | 0,2 - 1,0 | 20 - 100 ^{e)} |
| Milchsäure | 0,5 - 2 | 45 - 180 ^{f)} |

| | | |
|---|---|---|
| ^{a}) Konzentration in der Maische [5 - 20 % TM in der Maische]; ^{b}) Bezogen auf HNO₃ 65 % [65-prozentige Lösung]; ^{c)} Bezogen auf H₂SO₄ 98 % [98-prozentige Lösung] ; ^{d)} Bezogen auf HCl 37 % [37-prozentige Lösung]; ^{e)} Bezogen auf H₃PO₄ 85 % [85-prozentige Lösung]. ^{f)} Bezogen auf Milchsäure 90 % [90-prozentige Lösung]. | | |

Zur Gewinnung von Glukose für weitere Fermentationsprozesse erfolgt in einem zweiten Verfahrensschritt ein enzymatischer Aufschluss der thermisch behandelten schwer abbaubaren, lignifizierten Biomasse mit einem Gemisch aus cellulolytischen Enzymen. Das Gemisch besteht vorzugsweise aus Cellulosen, die einen Abbau von Hemicellulosen und Cellulosen in pflanzlichem Biomassegewebe bewirken. Biomasseballen, die über das zweistufige Verfahren aufgeschlossen wurden, zeigten eine hohe Aufschlusseffizienz, was über die Qualifizierung der freigesetzten Monosaccharide Xylose und Glucose ermittelt werden kann.

Vorzugsweise werden zum enzymatischen Aufschluss technische Cellulasen mit den folgenden Aktivitätsbereichen eingesetzt:

| | | |
|---|---|---|
| C1-Cellulase [Exocellulase] | 250 -1.000 | U/g oder U/ml |
| Endo-Cellulase | 25.000 -100.000 | U/g oder U/ml |
| β-Glucosidase | 50 -.300 | U/g oder U/ml |

Der Einsatzbereich von den Cellulasen beträgt vorzugsweise zwischen 0,5 g (bzw. ml) bis 10 g (bzw. ml) pro kg organischer Trockenmasse. Der pH-Wert der enzymatischen Behandlung erfolgt vorzugsweise bei einem Säuregrad (pH) von 3,0 bis 6,0, vorzugsweise bei einem pH zwischen 4 und 5. Der Temperaturbereich der enzymatischen Behandlung erfolgt vorzugsweise bei einer Temperatur zwischen 35° bis 65° C, vorzugsweise zwischen 45° und 60°C.

Sofern die zur Verfügung stehenden Biomassen in lignifizierter Form noch nicht in Ballenform vorliegen, können sie vor der Säurebehandlung bei Schritt a. in Ballenform gepresst werden. Die hohe Dichte der zu Ballen gepressten Biomasse ergibt eine höhere Hydrolysatmenge zur Weiterverarbeitung zu Industrieprodukten bzw. hydrolysierte Biomassemenge für eine anschließende Fermentation.

Vorzugsweise sind die Biomasseballen ausgewählt aus der Gruppe bestehend aus Strohballen, Heuballen, Helophytenballen (z.B. Miscanthusballen, Schilfballen) und Ballen sonstiger getrockneter Gräser oder Pflanzen.

Das erfindungsgemäße Aufschlussverfahren ermöglicht eine effiziente Weiterverarbeitung der Hydrolysate zu den jeweiligen Produkten, beispielsweise zur Herstellung von Zuckern, Zuckerersatzstoffen, Alkoholen oder Biopolymeren. Die hydrolysierte Biomasse kann zur Erzeugung von Biogas bei beträchtlich gesteigertem Biogasertrag eingesetzt werden.

Über die rein energetische Nutzung hinaus bietet der hydrothermale Aufschluss entsprechend dem erfindungsgemäßen Verfahren die zusätzliche Option einer stofflichen Nutzung durch die fermentative Gewinnung von hochwertigen Produkten für die Nahrungsmittelindustrie und die Biopolymerproduktion. Die mit Monosacchariden angereicherten Hydrolysate dienen hierbei als Substrate für verschiedene biotechnologische Konversionen. So lassen sich durch die Weiterverarbeitung der über das erfindungsgemäße Aufschlussverfahren gewonnenen Hydrolysate Zucker, Zuckerersatzstoffe, Alkohole oder Biopolymere herstellen. Die biotechnologische Produktion erfolgt auf der Basis von Xylose mit Hefen der Gattung *Candida.* Bei Fermentationsexperimenten mit Strohhydrolysaten können Umsatzraten von > 70 % (Xylose/Xylitol [g/g]) erzielt werden. Auf dieser Basis ergibt sich ein Ertragspotential von etwa 125 kg Xylitol je Tonne Stroh und damit ein erhebliches zusätzliches Wertschöpfungspotential. Ferner lassen sich auch Polyhydroxybuttersäure (PHB) als Alternative zu petrobasierten Kunststoffen wie Polypropylen oder Polyethylen herstellen. Die Kombination aus stofflicher und energetischer Nutzung bietet hierbei erhebliche Synergiepotentiale. Die im Rahmen der Biogasproduktion bereitgestellte Abwärme kann für die thermischen Aufschlüsse eingesetzt werden, die Reststoffe der Fermentationen werden wiederum der Biogasanlage zugeführt. Die Herstellung von Xylitol als Nebenprodukt des erfindungsgemäßen Aufschlussverfahrens sowie die Weiterverarbeitung der Hydrolysate zur Herstellung von Industrieprodukten, wie z.B. Polyhydroxybuttersäure (PHB), ist besonders bevorzugt. Die Erfindung umfasst daher auch Verfahren zur Herstellung solcher Zielprodukte unter Anwendung einer hier beschriebenen Vorrichtung oder des erfindungsgemäßen Aufschlussverfahrens.

Ein Vorteil des erfindungsgemäßen Verfahrens liegt in dem hohen Aufschlussgrad und der damit verbundenen Freisetzung von Stoffen im Hydrolysat, die zur Weiterverarbeitung genutzt werden können. Ferner wird der Biogasertrag erheblich gesteigert. Das Verfahren ermöglicht eine weitgehend strukturelle Desintegration schwer abbaubarer, lignifizierter Biomassen. Das erfindungsgemäße Aufschlussverfahren ist somit als Vorbehandlungsverfahren vor der eigentlichen Fermentation dieser Biomassen einsetzbar.

Der hydrothermale Aufschluss über das zweistufige Verfahren bietet insbesondere das Potential, lignifizierte Biomassen wie Stroh oder Heu für eine Biogasproduktion verfügbar zu machen. Bereits bei Temperaturen von 150°C kann eine weitgehende Hydrolyse der Hemicellulosefraktion erreicht werden. Darüber hinaus bedingt das Verfahren eine wesentlich verbesserte Abbaubarkeit des Celluloseanteils. Im Ergebnis wird die Vergärbarkeit des Substrates signifikant gesteigert. Im Vergleich zu unbehandeltem Stroh konnte eine Steigerung des Biogasertrages von bis zu 60 % demonstriert werden. Neben dem erhöhten Abbaugrad ergibt sich auch eine deutlich gesteigerte Abbaugeschwindigkeit, was in einer Reduktion der notwendigen hydraulischen Verweilzeit resultiert. Durch die weitgehende strukturelle Desintegration des Substrates ergeben sich zudem verfahrenstechnische Vorteile. So lässt sich das aufgeschlossene Substrat mit konventioneller Fördertechnik in den Biogasfermenter einbringen, und es tritt keine ausgeprägte Schwimmdeckenbildung auf. Aufgrund des Stickstoffdefizits und weiterer fehlender Nähr- und Spurenstoffe können Co-Substrate wie beispielsweise Rindergülle eingesetzt werden. Das erfindungsgemäße Verfahren ermöglicht eine alternative Nutzung von Ernteresten wie Stroh oder Heu und ist daher für die Biogaserzeugung besonders vorteilhaft.

### Kurze Beschreibung der Zeichnungen:

Die Erfindung wird in den nachfolgenden Beispielen und Figuren näher erläutert. Es zeigen
- Fig. 1: eine Ausführungsform der erfindungsgemäßen Vorrichtung zum hydrothermalen Aufschluss von getrockneten, verholzten Lignocellulosehaltigen Biomassen,
- Fig. 2: die Xyloseausbeute bei Weizenstroh in Abhängigkeit von Aufschlusstemperatur und Säurekonzentrationen,
- Fig. 3: den Xyloseertrag in Abhängigkeit von unterschiedlichen Behandlungstemperaturen bei Weizenstroh,
- Fig. 4: die Zuckerkonzentration nach enzymatischer Hydrolyse,
- Fig. 5: die Summe der organischen Säuren der Vergleichsansätze bei der Vorhydrolyse,
- Fig. 6: einen vergleichenden dynamischen Gärtest mit TDH- und unbehandeltem Weizenstroh,
- Fig. 7: einen vergleichenden statischen Gärtest mit TDH- und unbehandeltem Weizenstroh.

### Wege zur Ausführung der Erfindung und gewerbliche Verwertbarkeit:

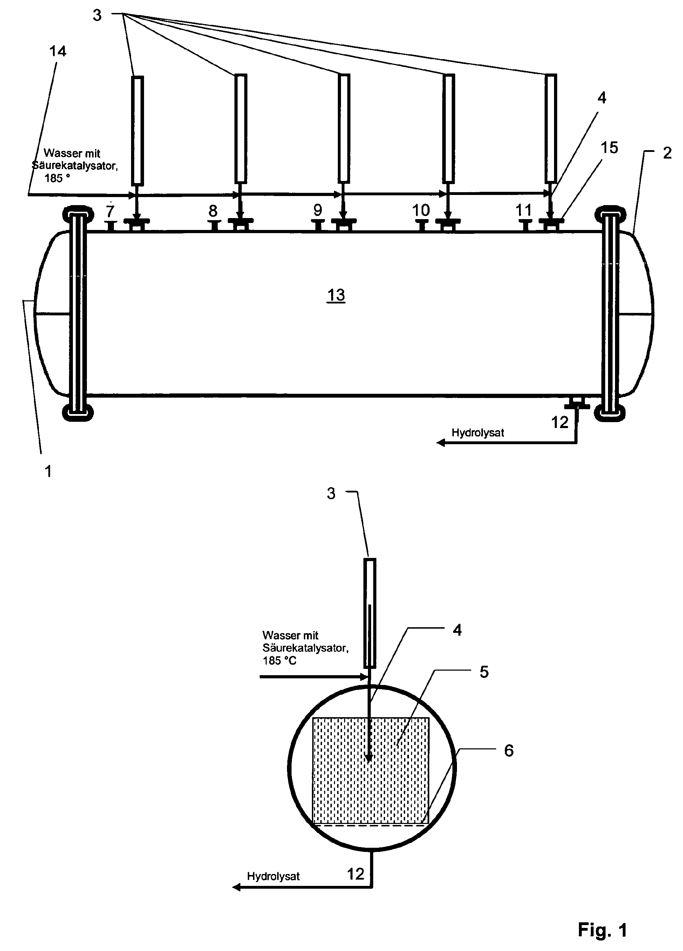

In Fig. 1 ist eine Ausführungsform einer erfindungsgemäßen Vorrichtung zum hydrothermalen Aufschluss stark abbaubarer, lignifizierter Biomassen gezeigt. Die Vorrichtung besteht aus einem Aufschlussbehälter 13, der an der Stirnseite eine Beschickungsöffnung 1 sowie auf der gegenüberliegenden Stirnseite eine Entleerungsöffnung 2 für die behandelten Biomassenballen 5 besitzt. Die Biomasseballen 5 (z.B. Strohballen in Form von Rest- oder Wickelballen) werden über die Beschickungsöffnung 1 in das Innere des Aufschlussbehälters 13 auf ein Sieb- und Führungselement 6 überführt. Danach wird die Beschickungsöffnung 1 geschlossen. Über Füllstutzen 15 wird ein Säure/Wassergemisch über eine Zuführleitung 14 in das Innere des Aufschlussbehälters 13 geleitet. Zur Injektion des Säure/Wassergemisches sind Injektionseinrichtungen 4 vorgesehen, die über Führungszylinder 3 bewegbar ausgestaltet sind. Die Injektionseinrichtungen 4 sind in der gezeigten Ausführungsform als Einstechlanzen ausgebildet und werden zur Injektion des vorgewärmten Säure/Wassergemisches von der Oberseite des Aufschlussbehälters 13 vertikal von oben nach unten geführt, sodass die Spitzen der Injektionseinrichtungen 4 in die Biomassenballen 5 zur direkten Injektion des Säure/Wassergemisches eingeschoben werden.

Das Säure/Wassergemisch wird über eine Vorheizeinrichtung vorgewärmt, vorzugsweise auf eine Temperatur von 180°C, sodass die Temperatur zum thermalen Aufschluss im Behälterinneren ca. 150°C beträgt.

Im Bereich des Behälterbodens befindet sich das Sieb- und Führungselement 6, das neben der Aufnahme der einzelnen Biomasseballen zur Abtrennung des flüssigen Hydrolysats von der festen Biomasse dient. Das so gewonnene Hydrolysat wird über einen Hydrolysatablauf 12 im Bereich des Behälterbodens zur Weiterverarbeitung (beispielsweise zur Herstellung von Zuckern, Zuckerersatzstoffen, Alkoholen oder Biopolymeren) abgelassen.

Die thermische Behandlung unter Zusatz mineralischer Säuren und/oder organischer Säuren erfolgt vorzugsweise bei Temperaturen zwischen 140° und 160°C für eine Zeitdauer zwischen ein und zwei Stunden. Mineralische Säuren sind besonders bevorzugt.

Die anschließende enzymatische Behandlung kann entweder in demselben Behälter oder alternativ in einem weiteren Reaktionsgefäß erfolgen. Die enzymatische Behandlung dauert etwa 1 bis 2 Tage und findet bei Temperaturen zwischen 35° und 65°C, vorzugsweise zwischen 45° und 60°C und einem pH-Bereich von 3,0 bis 6,0, vorzugsweise zwischen 4 und 5 statt. In diesem thermophilen Bereich haben die eingesetzten technischen Cellulasen ihre höchste Aktivität.

Die Vorwärmung des Wassers mit dem darin enthaltenen Säurekatalysator erfolgt vorzugsweise über eine Vorheizeinrichtung, die vor der Zuführleitung 14 angeordnet ist.

Die gezeigte Einrichtung besitzt ferner Mittel zur Temperaturüberwachung 7 und/oder Drucküberwachung 8 und/oder pH-Überwachung 9 und/oder ein Sicherheitsventil 10 und/oder einen Dampfauslass 11, um die Reaktionsbedingungen zu überwachen und ggf. zu steuern.

### Beispiele

### 1. Thermischer Aufschluss von Stroh

Die Untersuchungen zum hydrothermalen Aufschluss von Stroh erfolgten zunächst in einer speziellen Druckmikrowelle. Die Aufschlusseffizienz wurde über die Quantifizierung der freigesetzten Monosaccharide Xylose und Glucose mittels HPLC ermittelt. Als Substrate kamen verschiedene Strohsorten zum Einsatz, die nachfolgend dargestellten Ergebnisse beziehen sich auf Weizenstroh mit einem Cellulosegehalt von ca. 46 % sowie einem Hemicellulose- und Ligninanteil von jeweils ca. 20 % (bezogen auf die Trockenmasse).

Zur Absenkung des notwendigen Temperaturniveaus wurden den Ansätzen Salpetersäure in einem Konzentrationsbereich zwischen 0,05 und 0,4 % (v/v) zugegeben. Hinsichtlich der Xyloseausbeute zeigte sich deutlich der Einfluss der zugesetzten Säure auf die Aufschlusseffizienz (siehe **Fig. 1**). Bei Säurekonzentrationen bis ca. 0,06 % lagen die Xylosekonzentrationen unabhängig von der Aufschlusstemperatur bei maximal 1 % der Trockenmasse. Die Maxima wurden bei den Aufschlusstemperaturen 150 und 160°C im Konzentrationsbereich zwischen 0,2 und 0,3 % HNO₃ erreicht. Hierbei wurden Xyloseausbeuten von knapp über 20 % der Trockenmasse erzielt.

Bei den Aufschlusstemperaturen 130 und 140°C wurden die Maximalausbeuten bei der höchsten eingesetzten Säurekonzentration von ca. 0,4 % HNO₃ erreicht. Die Ansätze mit den höheren Aufschlusstemperaturen zeigten hier bereits einen deutlichen Rückgang der Xylosekonzentration bei einer gleichzeitig starken Zunahme der Furfural-Konzentrationen.

Die Glucoseausbeuten lagen erwartungsgemäß deutlich niedriger als bei der Xylose. Dies ist auf die sehr stabile Struktur der Cellulosefasern zurückzuführen, sodass hohe Temperaturen und/oder ein starker Säureeinsatz nötig ist/sind, um die Polymere in einem hohen Umfang zu hydrolysieren. Glucosekonzentrationen oberhalb von 5 % der Trockenmasse waren daher nur bei höheren Aufschlusstemperaturen in Verbindung mit verstärktem Säureeinsatz zu beobachten. Die Maximalausbeute beim Weizenstroh lag bei ca. 9 % der Trockenmasse.

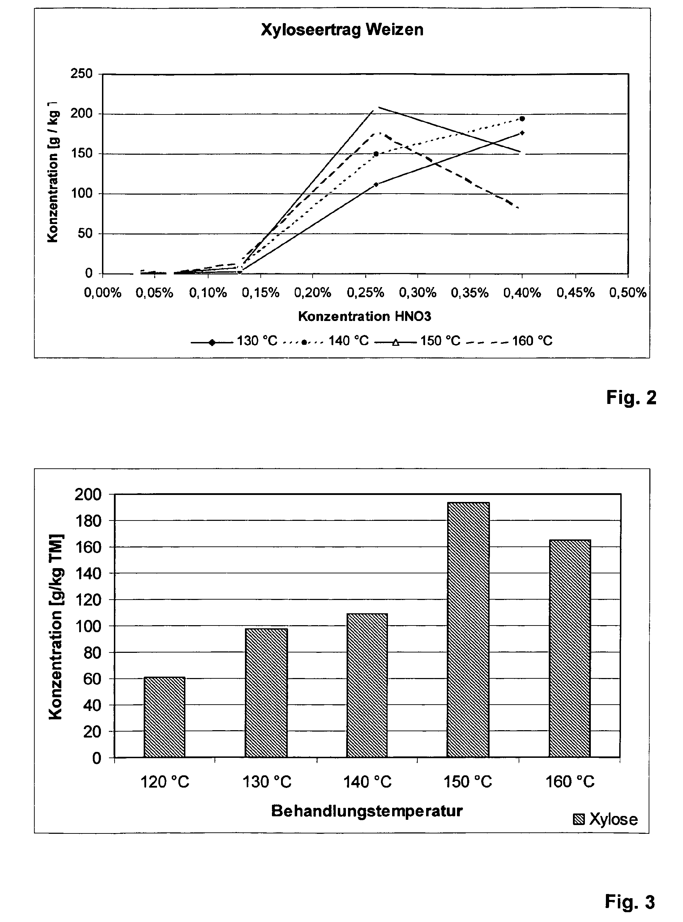

Im Anschluss an die Untersuchungen im Labormaßstab erfolgte ein Upscaling des Verfahrens in einen Technikumsmaßstab. Hierzu wurde ein TDH-Aufschlussreaktor mit einem Arbeitsvolumen von 100 I eingesetzt. Bei den Versuchen wurde überprüft, inwieweit sich die Ergebnisse der Mikrowellenaufschlüsse auf den Technikumsmaßstab übertragen lassen. Für die Serienaufschlüsse wurden jeweils 1,5 Kilogramm Weizenstroh mit einer Wasservorlage von 10 I für 1 Stunde bei Temperaturen zwischen 120 und 160°C behandelt. Die Xyloseausbeuten im Technikumsmaßstab erreichten nahezu das Niveau der Mikrowellenaufschlüsse, eine Ausnahme zeigte sich lediglich bei 140°C wo ein signifikant niedrigerer Ertrag erzielt wurde (siehe **Fig. 2**). Die höchste Ausbeute zeigte sich bei 150°C mit einem Xyloseertrag von ca. 19 %; dieser Wert repräsentierte den im Rahmen der Untersuchungen ermittelten Maximalertrag.

Die weitere Erhöhung der Behandlungstemperatur auf 160°C resultierte in einen Rückgang des Xyloseertrages auf rund 16,5 %. Ähnliche Effekte hatten sich bereits im Rahmen der Voruntersuchungen in der Druckmikrowelle gezeigt und lassen sich durch eine verstärkte Furfuralbildung erklären. Die Untersuchungsergebnisse zeigen, dass sich die Hemicellulosefraktion des Strohs unter optimierten Aufschlussbedingungen bei einer Behandlungstemperatur von 150°C nahezu vollständig hydrolysieren lässt. Eine Hydrolyse der Cellulosefraktion findet unter diesen Bedingungen nur eingeschränkt statt. Es wurde deshalb in einem weiteren Verfahrensschritt untersucht, in welchen Maße Cellulosefasern durch die hydrothermale Vorbehandlung in der Ligninmatrix freigelegt wurden und damit besser für eine enzymatische Hydrolyse verfügbar sind.

Entsprechende Untersuchungen von TDH-behandelten und unbehandeltem Stroh wurden mit kommerziell erhältlichen cellulolytischen Enzymen durchgeführt. Die Ansätze (TS-Gehalt der Maische ca. 4 %; pH 4,5) wurden für 20 h mit zwei unterschiedlichen Enzymkonzentrationen (1 und 3 % bezogen auf die Trockenmasse) bei 50°C inkubiert. **Figur 3** zeigt den deutlich erhöhten Abbaugrad bei den TDH-behandelten Ansätzen.

Bezogen auf die Gesamtzucker (Summe Glucose / Cellobiose dargestellt als Glucoseäquivalent) liegt die Steigerung bei einer geringeren Enzymkonzentrationen bei ca. 100 % während bei einem stärkeren Enzymeinsatz eine ca. 4-fache höhere Konzentration vorlag als im unbehandelten Vergleichsansatz. Der geringe Effekt einer deutlichen Erhöhung der Enzymkonzentration beim unbehandelten Stroh deutet darauf hin, dass strukturell intakte und in die Liginmatrix eingebettete Cellulosefasern nur im sehr geringen Maße einem enzymatischen Abbau unterworfen sind.

### 2. Effekte des thermischen Aufschlusses auf die Vergärbarkeit und den Biogasertrag

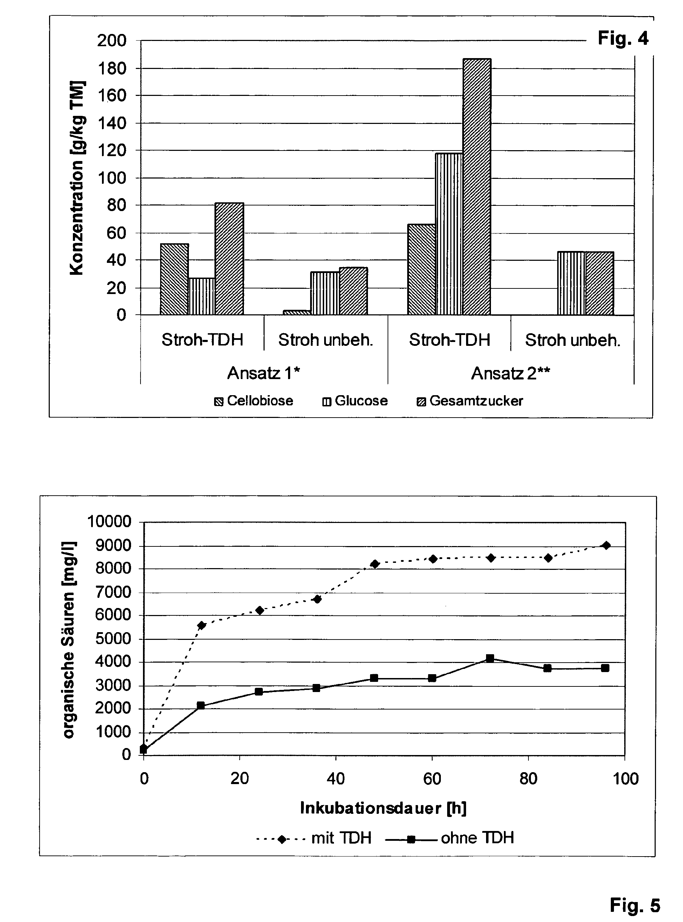

Zur vergleichenden Untersuchung der Vergärbarkeit von TDH-behandelten und unbehandelten Stroh können verschiedene Versuchsansätze angewandt werden. Beim Versäuerungs- oder Hydrolyseansatz werden die zu untersuchenden Substrate bei sauren Bedingungen (Start pH-Wert <6) über mehrere Tage bei Sauerstoffabschluss inkubiert. Das über den Versuchszeitraum mittels HPLC aufgenommene Versäuerungsspektrum sowie die Summe der gebildeten Säuren geben einen Aufschluss über die Vergärbarkeit des jeweiligen Substrats. Die in **Figur 4** dargestellte Säurebildung der Vergleichsansätze (Weizenstroh unbehandelt, Weizenstroh TDHbehandelt) zeigt, dass die Versäuerung im TDH-Ansatz schneller und in einen deutlich stärkeren Umfang stattfindet. Zum Ende des Inkubationszeitraums lag die Summe der organischen Säuren im TDH-Ansatz um mehr als 100 % über dem unbehandelten Vergleichsansatz.

Die im Rahmen der Untersuchungen festgestellte schnellere und insbesondere wesentlich stärkere Versäuerung der TDH-behandelten Ansätze deutet auf eine signifikant verbesserte Vergärbarkeit infolge der hydrothermalen Behandlung hin.

Im Anschluss an die Versäuerungsexperimente wurden vergleichende dynamische Gärtest durchgeführt um den Effekt der hydrothermalen Behandlung auf den spezifischen Biogasertrag des Substrats zu überprüfen. Die Untersuchungen wurden mit Pilot-Biogasfermentern (100 I Durchflussfermenter) durchgeführt, die eine praxisnahe Untersuchung von Substraten für den Biogasprozess erlauben. In einem Parallelansatz mit zwei baugleichen Fermentern wurde die Biogasproduktion von TDH-behandelten Weizenstroh (1 h bei 140°C) im Vergleich zu unbehandelten Stroh über einen Zeitraum von 5 Wochen verfolgt. Nach Abschluss der Anfahrphase erreichte der mit unbehandeltem Stroh betriebene Fermenter (F2) im Durchschnitt eine Biogasproduktion von 308 Nl / kg oTS bei einem mittleren Methangehalt von 50 %. Beim mit TDH-Stroh betriebenen Vergleichsfermenter (F1) lag die durchschnittliche Produktion im Betrachtungszeitraum bei 490 Nl / kg oTS bei einem Methangehalt von ebenfalls ca. 50 %. Somit konnte auf Basis des dynamischen Gärtests eine Steigerung des spezifischen Biogasertrags infolge des hydrothermalen Aufschlusses um ca. 60 % demonstriert werden (siehe **Fig. 5**).

Als eine kritische Größe bei der Monovergärung von Stroh erwies sich in beiden Versuchsansätzen der pH-Wert. Aufgrund des Stickstoffdefizits von Stroh liegen in den Fermentern niedrige Ammoniumkonzentrationen und damit eine geringe Pufferkapazität vor. Daraus resultiert bei einer Monofermentation von Stroh ein erhöhtes Risiko einer Versäuerung mit deutlichem pH-Abfall. In der Praxis lässt sich dieses Problem durch den Einsatz eines stickstoffreichen Co-Substrats (z.B. Gülle) beheben. Neben der erheblichen Steigerung des spezifischen Biogasertrags ergeben sich durch die hydrothermale Behandlung weitere verfahrenstechnische Vorteile. Zum einen bringt der Einsatz von unbehandeltem Stroh erhebliche Förderprobleme mit sich, da das Substrat dazu neigt sich um die Förderschnecke zu wickeln. Weiterhin kommt es im Fermenter sehr schnell zu einer ausgeprägten Schwimmdeckenbildung. Beide Probleme treten bei hydrothermal behandeltem Stroh nicht auf.

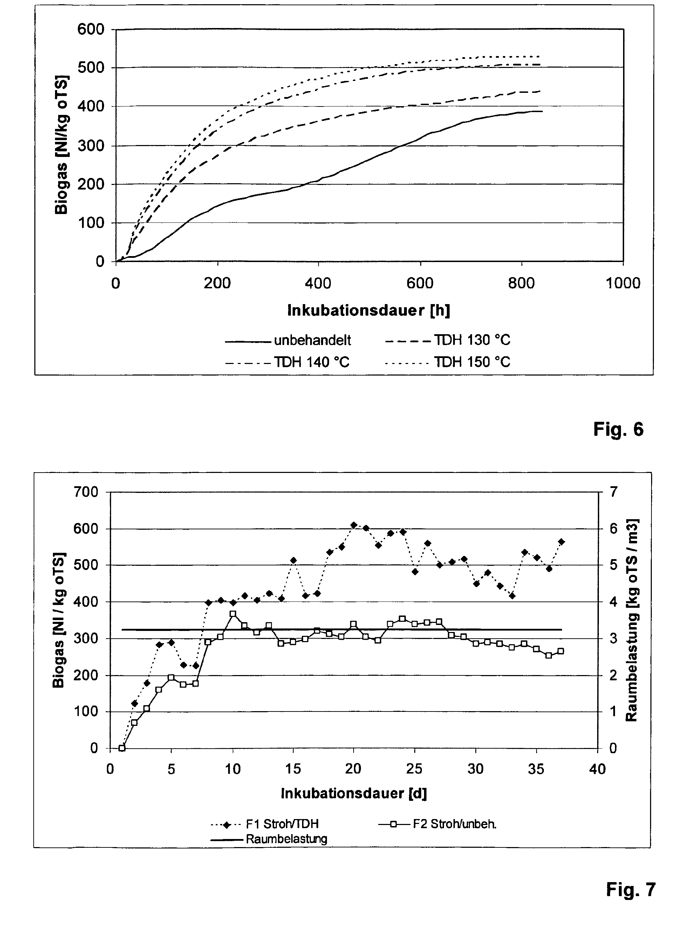

Um den Einfluss unterschiedlicher Behandlungstemperaturen auf das Ausgasungsverhalten näher zu untersuchen, wurden im Anschluss statische Gärtests durchgeführt. Als Vergleichsansatz wurde parallel ein Ansatz mit unbehandeltem Stroh untersucht. Dieser zeigte einen langsamen zweistufigen Anstieg der Gassummenkurven. Nach einem steileren Anstieg innerhalb der ersten 200 h Inkubationsdauer zeigte sich zunächst ein Abflachen, im Anschluss war ein erneuter Anstieg feststellbar bis die Summenkurve ab ca. 700 h zunehmend in den Sättigungsbereich überging (siehe **Fig. 6**).

Insgesamt wurde eine Biogasmenge von 386 Nl / kg oTS für das unbehandelte Weizenstroh ermittelt. Dieser Ertrag lag damit deutlich oberhalb der Produktion die im Rahmen des dynamischen Gärtests ermittelt wurde (308 Nl / kg oTS). Allerdings wurde dieser Wert erst nach einer Inkubationszeit von 840 h erreicht. Innerhalb der ersten 240 h Inkubationszeit waren hiervon erst rund 41 % gebildet und nach 480 h ca. 65 %. Im Vergleich hierzu zeigte sich bei dem TDH-behandelten Stroh ein deutlich steilerer und kontinuierlicher Verlauf der Gassummenkurven. Der insgesamt erzielte Mehrertrag im Vergleich zum unbehandelten Stroh lag zwischen 13 und 36 %. Deutlicher wird der Unterschied beim Vergleich der relativ zur Gesamtproduktion erreichten Werte nach den Inkubationszeiten 240 h und 480 h. Der Ansatz TDH 130° C hatte hier bereits rund 68 % bzw. 87 % der Gesamtmenge produziert. Einen noch höheren Anteil wiesen die Ansätze mit 150°C und 160°C Behandlungstemperatur auf. Hier waren nach 240 h 73 und 76 % sowie nach 480 h bereits 92 bzw. 94 % der gesamten Gasmenge gebildet. Diese Werte zeigen den deutlich schnelleren Abbau des TDH behandelten Materials.

### 3. Kombinierte stoffliche und energetische Nutzung

Über die rein energetische Nutzung hinaus bietet der hydrothermale Aufschluss von Stroh die zusätzliche Option einer stofflichen Nutzung durch die fermentative Gewinnung von hochwertigen Produkten für die Nahrungsmittelindustrie und die Biopolymerproduktion. Die mit Monosacchariden angereicherten Hydrolysate dienen hierbei als Substrate für verschiedene biotechnologische Konversionen. Im Rahmen des dargestellten Forschungsprojektes konnte die biotechnologische Gewinnung des hochpreisigen Zuckerersatzstoffes Xylitol auf der Basis von Strohhydrolysaten demonstriert werden. Der Zuckeralkohol Xylitol wird für spezielle Anwendungen in der Nahrungsmittelproduktion und im Pharmabereich eingesetzt und erzielt Marktpreise im Bereich von 4.000 €/t. Die biotechnologische Produktion erfolgt auf der Basis von Xylose mit Hefen der Gattung *Candida.* Bei Fermentationsexperimenten mit Strohhydrolysaten konnten Umsatzraten von > 70 % (Xylose/Xylitol [g/g] erzielt werden. Auf dieser Basis ergibt sich ein Ertragspotential von rund 125 kg Xylitol je Tonne Stroh und damit eine erhebliches zusätzliches Wertschöpfungspotential. Gegenstand aktuell laufender Forschungsarbeiten ist die fermentative Gewinnung des Biopolymers Polyhydroxybuttersäure (PHB) als Alternative zu petrobasierten Kunststoffen wie Polypropxlen oder Polyethylen. Die Kombination aus stofflicher und energetischer Nutzung bietet hierbei erhebliche Synergiepotentiale. Die im Rahmen der Biogasproduktion bereitgestellte Abwärme kann für die thermischen Aufschlüsse eingesetzt werden, die Reststoffe der Fermentationen werden wiederum der Biogasanlage zugeführt.

## Patentansprüche

1. Vorrichtung zum hydrothermalen Aufschluss von getrockneten, verholzten Lignocellulose-haltigen Biomassen, insbesondere solchen, die in Ballenform vorliegen, bestehend aus einem Aufschlussbehälter (13) zur Aufnahme von Biomasseballen (5), mit einer Beschickungsöffnung (1) für die Biomassenballen (5) sowie einer Entleerungsöffnung (2) für die Entfernung der behandelten Biomasse aus dem Aufschlussbehälter (13), wobei über eine Zuführleitung (14) ein vorgeheiztes Säure/Wassergemisch über Füllstutzen (15) in das Innere des Aufschlussbehälters (13) leitbar ist und das beim hydrolytischen Aufschluss entstehende Hydrolysat über einen Hydrolysatablauf (12) aus dem Aufschlussbehälter (13) abführbar ist, **dadurch gekennzeichnet, dass** in dem Aufschlussbehälter (13) wenigstens eine stationäre oder bewegliche Injektionseinrichtung (4) für das vorgeheizte Säure/Wassergemisch und im Bodenbereich des Aufschlussbehälters (13) ein Sieb- und Führungselement (6) zur Abtrennung des flüssigen Hydrolysats von der festen Biomasse angeordnet ist.

2. Vorrichtung nach Anspruch 1, **dadurch gekennzeichnet, dass** die Injektionseinrichtung (4) als Einstechlanze ausgebildet und über einen Führungszylinder (3) von der Oberseite des Aufschlussbehälters (13) in den Biomassenballen (5) zur Injektion des Säure/Wassergemisches in den Ballen einschiebbar ist.

3. Vorrichtung nach einem der Ansprüche 1 bis 2, **dadurch gekennzeichnet, dass** zur Vorwärmung des Säure/Wassergemisches eine Vorheizeinrichtung vorgesehen ist.

4. Vorrichtung nach einem der Ansprüche 1 bis 3, **dadurch gekennzeichnet, dass** diese Mittel zur Temperaturüberwachung (7) und/oder Drucküberwachung (8) und/oder pH-Überwachung (9) und/oder ein Sicherheitsventil (10) und/oder einen Dampfauslass (11) umfasst.

5. Verfahren zum hydrothermalen Aufschluss von getrockneten, verholzten Lignocellulose-haltigen Biomassen, die in Ballenform vorliegen, bestehend aus dem Schritt:
a. Inkubation der Lignocellulose-haltigen verholzten Biomasse bei einer Temperatur zwischen 120° und 220°C mit einem vorgeheizten Säure/Wassergemisch, bestehend aus mindestens einer mineralischen und/oder organischen Säure.

6. Verfahren nach Anspruch 5, **dadurch gekennzeichnet, dass** nach dem hydrothermalen Säureaufschluss im Schritt a. eine anschließende enzymatische Behandlung gemäß Schritt b. erfolgt:
b. Enzymatischer Aufschluss der in Schritt a. säurebehandelten Biomasse mit einem Gemisch aus cellulolytischen Enzymen bei einer Temperatur zwischen 35 und 65°C und einem Säuregrad (pH) im Bereich von 3-6.

7. Verfahren nach Anspruch 5 oder 6, **dadurch gekennzeichnet, dass** die Inkubation des Schrittes a. bei einer Temperatur zwischen 140° und 160°C erfolgt.

8. Verfahren nach einem der Ansprüche 5 bis 7, **dadurch gekennzeichnet, dass** das Säure/Wassergemisch eine mineralische Säure, ausgewählt aus der Gruppe, bestehend aus Salpetersäure, Schwefelsäure, Salzsäure, Phosphorsäure, Milchsäure enthält.

9. Verfahren nach einem der Ansprüche 5 bis 8, **dadurch gekennzeichnet, dass** die Biomasse vor der Säurebehandlung bei Schritt a. in Ballenform gepresst wird.

10. Verfahren nach einem der Ansprüche 5 bis 9, **dadurch gekennzeichnet, dass** die Biomasse ausgewählt ist aus der Gruppe, bestehend aus Strohballen, Heuballen, Helophytenballen (z.B. Miscanthusballen, Schilfballen) und Ballen sonstiger getrockneter Gräser oder Pflanzen.

## Claims

1. Device for hydrothermally pulping dried, lignified lignocellulose-containing biomasses, in particular those which are in bales, consisting of a pulp container (13) for receiving biomass bales (5), comprising a loading opening (1) for the biomass bales (5) and an emptying opening (2) for removing the treated biomass from the pulp container (13), it being possible to convey a preheated acid-water mixture via a feed line (14) into the interior of the pulp container (13) by means of fill nozzles (15) and it being possible to convey the hydrolysate produced during the hydrolytic pulping out of the pulp container (13) via a hydrolysate drain (12), **characterised in that** at least one stationary or movable injection means (4) for the preheated acid-water mixture is arranged in the pulp container (13) and a screen-and-guide element (6) for separating the liquid hydrolysate from the solid biomass is arranged in the floor region of the pulp container (13).

2. Device according to claim 1, **characterised in that** the injection means (4) is in the form of a plunge lance and can be pushed into the biomass bales (5) from the top of the pulp container (13) by means of a guide cylinder (3) in order to inject the acid-water mixture into the bales.

3. Device according to either claim 1 or claim 2, **characterised in that** a preheating means is provided for preheating the acid-water mixture.

4. Device according to any of claims 1 to 3, **characterised in that** said device comprises means for monitoring temperature (7) and/or for monitoring pressure (8) and/or for monitoring pH (9), and/or a safety valve (10) and/or a steam outlet (11).

5. Method for hydrothermal pulping of dried, lignified lignocellulose-containing biomasses which are in bales, consisting of the step of:
a) incubating the lignocellulose-containing, lignified biomass at a temperature of between 120 and 220 °C using a preheated acid-water mixture consisting of at least one inorganic and/or organic acid.

6. Method according to claim 5, **characterised in that**, following the hydrothermal acid pulping in step a., a subsequent enzymatic treatment according to step b. takes place:
b. enzymatic pulping of the biomass, which is acid-treated in step a., using a mixture of cellulolytic enzymes at a temperature of between 35 and 65°C and at an acidity (pH) in the range of 3-6.

7. Method according to either claim 5 or claim 6, **characterised in that** the incubation of step a. takes place at a temperature of between 140 and 160°C.

8. Method according to any of claims 5 to 7, **characterised in that** the acid-water mixture contains an inorganic acid selected from the group consisting of nitric acid, sulfuric acid, hydrochloric acid, phosphoric acid, and lactic acid.

9. Method according to any of claims 5 to 8, **characterised in that** the biomass is pressed into bales prior to the acid treatment in step a.

10. Method according to any of claims 5 to 9, **characterised in that** the biomass is selected from the group consisting of straw bales, hay bales, helophyte bales (e.g. miscanthus bales, reed bales) and bales of other dried grasses or plants.

## Revendications

1. Dispositif de digestion hydrothermale de biomasses lignifiées séchées contenant de la lignocellulose, en particulier de biomasses qui sont présentes sous la forme de balles, constitué d'un récipient de digestion (13) destiné à recevoir des balles de biomasse (5), comprenant une ouverture de chargement (1) pour les balles de biomasse (5) ainsi qu'une ouverture de décharge (2) pour éliminer la biomasse traitée du récipient de digestion (13), un mélange acide/eau préchauffé pouvant être amené par une conduite d'alimentation (14) et par des tubulures de remplissage (15) à l'intérieur du récipient de digestion (13) et l'hydrolysat formé lors de la digestion hydrolytique pouvant être évacué du récipient de digestion (13) par une sortie d'hydrolysat (12), **caractérisé en ce qu'**au moins un dispositif d'injection (4) fixe ou mobile pour le mélange acide/eau préchauffé est disposé dans le récipient de digestion (13) et un élément de criblage et de guidage (6) pour séparer l'hydrolysat liquide de la biomasse solide est disposé dans la zone de fond du récipient de digestion (13).

2. Dispositif selon la revendication 1, **caractérisé en ce que** le dispositif d'injection (4) est réalisé sous la forme d'une sonde à piquer qui peut être insérée par un cylindre de guidage (3) depuis le côté supérieur du récipient de digestion (13) dans les balles de biomasse (5) pour injecter le mélange acide/eau dans les balles.

3. Dispositif selon l'une des revendications 1 à 2, **caractérisé en ce qu'**un dispositif de préchauffage est prévu pour préchauffer le mélange acide/eau.

4. Dispositif selon l'une des revendications 1 à 3, **caractérisé en ce que** celui-ci comprend des moyens de surveillance de la température (7) et/ou de surveillance de la pression (8) et/ou de surveillance du pH (9) et/ou une soupape de sécurité (10) et/ou une sortie de vapeur (11).

5. Procédé de digestion hydrothermale de biomasses lignifiées séchées contenant de la lignocellulose, en particulier de biomasses qui sont présentes sous la forme de balles, comprenant l'étape suivante :
a. incubation de la biomasse lignifiée contenant de la lignocellulose à une température comprise entre 120 et 220°C avec un mélange acide/eau préchauffé, composé d'au moins un acide minéral et/ou organique.

6. Procédé selon la revendication 5, **caractérisé en ce que** la digestion acide hydrothermale à l'étape a. est suivie d'un traitement enzymatique selon l'étape b. :
b. digestion enzymatique de la biomasse traitée à l'acide à l'étape a. par un mélange d'enzymes cellulolytiques à une température comprise entre 35 et 65°C et un degré d'acidité (pH) dans la plage comprise entre 3 et 6.

7. Procédé selon la revendication 5 ou 6, **caractérisé en ce que** l'incubation de l'étape a. est effectuée à une température comprise entre 140 et 160°C.

8. Procédé selon l'une des revendications 5 à 7, **caractérisé en ce que** le mélange acide/eau contient un acide minéral sélectionné dans le groupe constitué de l'acide nitrique, de l'acide sulfurique, de l'acide chlorhydrique, de l'acide phosphorique et de l'acide lactique.

9. Procédé selon l'une des revendications 5 à 8, **caractérisé en ce que** la biomasse est pressée en forme de balle avant le traitement à l'acide à l'étape a.

10. Procédé selon l'une des revendications 5 à 9, **caractérisé en ce que** la biomasse est sélectionnée dans le groupe constitué de balles de paille, balles de foin, balles d'hélophytes (p. ex. balles de miscanthus, balles de jonc) et balles d'autres graminées ou plantes séchées.
